# EUROPÄISCHE PATENTSCHRIFT

(11) **EP 1 298 440 B1**
(45) Veröffentlichungstag und Bekanntmachung des Hinweises auf die Patenterteilung: **14.02.2007**
(21) Anmeldenummer: 02450212.2
(22) Anmeldetag: 19.09.2002
(51) Int. Cl.: G01N 33/68, G01N 33/50, C12N 15/11

(54) **Funktionelles Screening für Transkriptionsfaktoren**
Functional screening for transcription factors
Criblage fonctionnel pour les facteurs de transcription

(30) Priorität: 27.09.2001 AT 15382001
(43) Veröffentlichungstag der Anmeldung: 02.04.2003
(73) Patentinhaber: BMT Medizinische Forschung und Entwicklung Gmbh, 1235 Wien (AT)
(72) Erfinder: Wiesner, Christoph, 1100 Wien (AT); Hoeth, Martina, 1050 Wien (AT); De Martin, Rainer, 1120 Wien (AT)
(74) Vertreter: Patentanwälte BARGER, PISO & PARTNER

(56) Entgegenhaltungen:
- WO-A-99/35282
- HERRING B PAUL ET AL: "Identification of Barx2B, a serum response factor-associated homeodomain protein" JOURNAL OF BIOLOGICAL CHEMISTRY, Bd. 276, Nr. 17, 27. April 2001 (2001-04-27), Seiten 14482-14489, XP002276017 ISSN: 0021-9258
- SAUVE FREDERIC ET AL: "CIA, a novel estrogen receptor coactivator with a bifunctional nuclear receptor interacting determinant" MOLECULAR AND CELLULAR BIOLOGY, Bd. 21, Nr. 1, Januar 2001 (2001-01), Seiten 343-353, XP002276018 ISSN: 0270-7306
- HE CHUAN HUA ET AL: "Identification of activating transcription factor 4 (ATF4) as an Nrf2-interacting protein: Implication for heme oxygenase-1 gene regulation" JOURNAL OF BIOLOGICAL CHEMISTRY, Bd. 276, Nr. 24, 15. Juni 2001 (2001-06-15), Seiten 20858-20865, XP002276019 ISSN: 0021-9258
- FIELDS S ET AL: "A NOVEL GENETIC SYSTEM TO DETECT PROTEIN-PROTEIN INTERACTIONS" NATURE, MACMILLAN JOURNALS LTD. LONDON, GB, Bd. 340, Nr. 6230, 20. Juli 1989 (1989-07-20), Seiten 245-246, XP000103144 ISSN: 0028-0836
- LI J J ET AL: "ISOLATION OF ORC6, A COMPONENT OF THE YEAST ORIGIN RECOGNITION COMPLEX BY A ONE-HYBRID SYSTEM" SCIENCE, AMERICAN ASSOCIATION FOR THE ADVANCEMENT OF SCIENCE,, US, Bd. 262, 17. Dezember 1993 (1993-12-17), Seiten 1870-1874, XP002067718 ISSN: 0036-8075
- WEI ZHENG ET AL: "Identification of a new sea urchin Ets protein, SpEts4, by yeast one-hybrid screening with the hatching enzyme promoter" MOLECULAR AND CELLULAR BIOLOGY, Bd. 19, Nr. 2, Februar 1999 (1999-02), Seiten 1271-1278, XP002276020 ISSN: 0270-7306

## Beschreibung

Die Erfindung betrifft ein Verfahren zur spezifischen Identifikation von Transkriptionsfaktoren und Transkriptionskoaktivatoren aus einem bestimmten Zelltyp.

Den aus dem Stand der Technik bekannten Dokumenten, Journal of Biological Chemistry (27-04-2001), 276(17), 14482-14489; Molecular and Cellular Biology (01-2001), 21(1), 343-353; Journal of Biological Chemistry (15-06-2001), 276(24), 20858-20865, ist gemeinsam, dass sie Identifizierung von Transkriptionsfaktoren, Co-Aktivatoren usw. beschreiben, aber ausgehend von einem bestimmten Faktor, für welchen diese Transkriptionsfaktoren, Co-Aktivatoren usw. dann auch spezifisch sind, z.B. Identifizierung von ATF4 ausgehend von Nrf2 als "bait".

In der WO-A-9935282 ist ein Verfahren beschrieben, dessen Ziel das Screening auf Modulatoren, insbesondere Inhibitoren, von Protein-Protein, gegebenenfalls Protein-DNA, Interaktionen ist, wobei zwei bestimmte, miteinander interagierende Proteine eingesetzt werden, und dann auf Modulation (Inhibition) dieser Interaktion gescreent wird.

Transkriptionsfaktoren (TF) steuern die Genexpression und sind somit regulatorische Schlüsselmoleküle für eine Vielzahl von Zellfunktionen, was sie zu wertvollen Ansatzpunkten für therapeutische Interventionen macht. Sie bestehen für gewöhnlich aus einer DNA-Bindungsdomäne (BD), einer Transaktivierungsdomäne (TA) und manchmal aus zusätzlichen Domänen wie z.B. einer Dimerisierungs- oder Ligandenbindungsdomäne. Die Genexpression erfolgt durch Bindung an sequenzspezifische Bindungsstellen in den jeweiligen Promotorregionen und Interaktion mit der basalen Transkriptionsmaschinerie.

Obgleich die funktionale Annotation von Sequenzen aus dem Human-Genom-Projekt und anderen Sequenzierungsprojekten insofern erfolgreich war, als eine Vielzahl von Proteindomänen identifiziert werden konnte, hat sich dies bei den TFs als schwierig erwiesen. Auch wenn einige DNA-Bindungs-Domänen (BDs), wie z.B. die Homöodomäne oder die Fork-Head-Domäne (Weigel et al., 1990; Miller et al., 1994), auf TFs hindeuten, so könnten andere, wie etwa Helix-Turn-Helix oder Leucin-Zipper, ebenfalls als Mediatoren für Protein-Protein-Interaktionen fungieren. Andererseits lässt die Definition von TA-Domänen sogar noch mehr zu wünschen übrig. Daher wurde ein genetisches Screening zur Identifizierung von TFs anhand ihrer TA-Eigenschaften entwickelt, gefolgt von einer Analyse im Hinblick auf das Vorhandensein einer potentiellen DNA-Bindungs-Domäne (BD).

Das Verfahren gemäß der Erfindung ist nun dadurch gekennzeichnet, dass eine cDNA-Bibliothek in einem Vektor generiert wird, welche Inserts als Fusionsproteine mit der Hefe Gal4 DNA Bindungsdomäne exprimiert.

### Identifikation von TFs

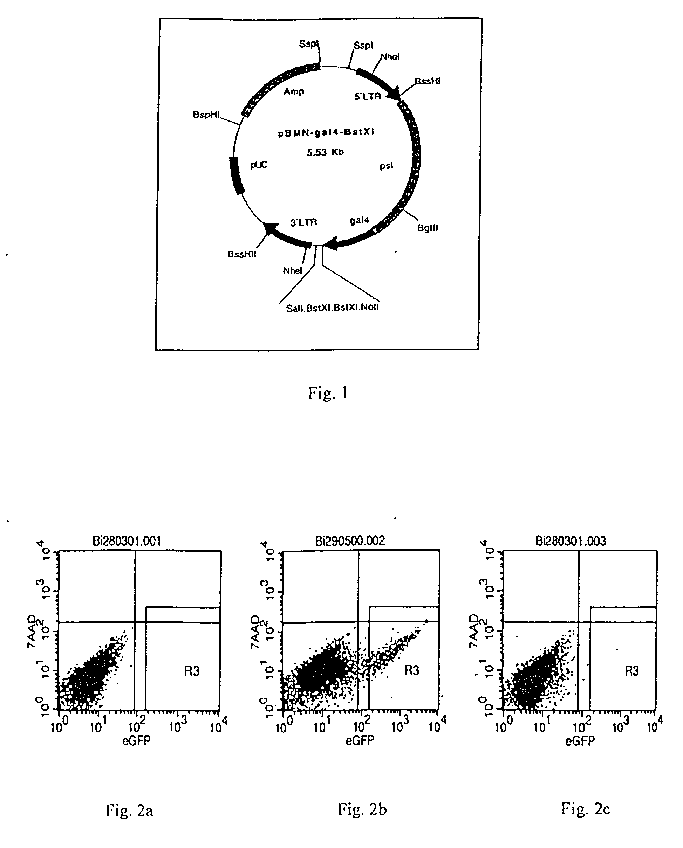

**Das "Mammalian Reverse One-Hybrid System".** Das klassische "Yeast-One-Hybrid-System" ist eine Methode zur Identifizierung von TFs, die an bekannte (multimerisierte) DNA-Bindungsstellen (Wei et al., 1999) binden. Dazu wird eine cDNA-Bibliothek verwendet, die Inserts als Fusionsgene mit der Gal4-TA-Domäne exprimiert, wodurch es zu Hefewachstum auf selektiven Medien kommt, falls das cDNA-Insert die DNA-Bindungsdomäne enthält, welche die jeweilige DNA-Sequenz erkennt. Im Gegensatz dazu wurde hier eine cDNA-Bibliothek in einem Vektor generiert, welche Inserts als Fusionsgene mit der Hefe-Gal4-DNA-Bindungsdomäne exprimiert (Fig. 1), und für Säugetierzellen adaptiert. Das Verfahren ist somit gekennzeichnet durch einen Plasmidvektor welcher cDNA Inserts als Fusionsproteine mit der Hefe Gal4 DNA Bindungdomäne exprimiert; weiters ist es gekennzeichnet durch eine dazu passende Reporterzelllinie, welche ein Gal4-abhängiges Promoter-Reportergen enthält, sodass cDNA Inserts, welche eine Transaktivierungs-Domäne enthalten, zusammen mit der Gal4 DNA Bindungsdomäne aus dem Vektor einen funktionellen Transkriptionsfaktor bilden, welcher die Expression des Reportergens bewirkt.

Es handelt sich somit um ein allgemeines Verfahren um Transkriptionsfaktoren unabhängig von ihrer DNA Bindungssequenz sowie Co-Aktivatoren zu identifizieren. Man spricht daher vom "Mammalian Reverse One-Hybrid-System". Das Verfahren unterscheidet sich somit vom Hefe One-Hybrid System unter anderem dadurch, dass bei Letzterem mittels einer bekannten DNA Sequenz spezifische Transkriptionsfaktoren gesucht werden welche ausschliesslich an jene vorgegebene Sequenz binden.

Fig. 1 zeigt eine Darstellung des retroviralen Vektors pBMN-Ga14-BStXI (abgekürzt: pBG4B). Es wurde ein retroviraler Vektor mit der Bezeichnung pBG4B konstruiert, welcher auf pBABE (Morgenstern und Land, 1990) basiert und die Hefe-Gal4-DNA-BD oberhalb eines Polylinkers mit zwei BstXI-Bindungsstellen und eingefügter cDNA aus zytokin-aktivierten Endothelzellen enthält. Nach Verpackung in retrovirale Partikel wurde die Bibliothek verwendet, um eine murine NIH3T3-basierende Reporter-Zelllinie, die mit einem Gal4-abhängigen EGFP-Reportergen (Enhanced Green Fluorescent Protein; Tsien 1998) stabil transfiziert wurde, zu transduzieren. cDNAs, welche für eine funktionelle TA-Domäne kodieren, aktivieren das Reportergen, und EGFP-positive Zellen werden mittels FACS-Analyse isoliert und expandiert, wodurch die Isolierung und Analyse der eingefügten Inserts möglich wird.

Das Screeningverfahren ist durch die folgenden Schritte gekennzeichnet:
1. Generierung einer Reporter-Zelllinie durch stabile Transfektion eines Gal4-abhängigen Promotor-EGFP-Reporterkonstrukts in NIH3T3-Zellen.
2. Isolierung der mRNA aus zytokin-aktivierten Endothelzellen
3. Synthese der cDNA
4. Ligation in pBMN-Gal4-BstXI unter Verwendung von BstXI-Adaptern
5. Transformation in elektrokompetente E. coli-Zellen
6. Präparation von Plasmid-DNA
7. Transfektion von Plasmid-DNA in Phoenix eco retrovirale Verpackungszellen
8. Sammeln des Retrovirus-Überstands und Infektion von NIH3T3-Gal4-Reporterzellen
9. Sortieren GFP-positiver Zellen mittels FACS-Analyse
10. Expansion der sortierten Zellen, Isolierung der mRNA und Isolierung von cDNA-Inserts durch RT-PCR, Sequenzierung

Dieses Konzept wurde zunächst mittels der NF-kB-p65-TA-Domäne (Schmitz et al., 1994) getestet, welche in den retroviralen Vektor als Positivkontrolle eingefügt wurde. Nach der retroviralen Transduktion konnten EGFP-positive Zellen mittels FACS-Analyse auf einfache Weise von den Kontrollzellen unterschieden werden, siehe Fig. 2a, 2b, 2c betreffend die FACS-Sortierung GFP-positiver Zellen, wobei sich Fig. 2a auf eine Negativkontrolle (nicht infizierte Reporterzellen) bezieht; Fig. 2b bezieht sich auf die Positivkontrolle (Reporterzellen transduziert mit NF-kB-p65-TA in pBMN-Gal4-BstXI; Fig. 2c bezieht sich auf mit der retroviralen Bibliothek transduzierte Reporterzellen. Es wurde festgestellt, dass ein Ergebnis von einer positiven pro 20.000 Zellen in der FACS-Analyse einer einzelnen Integration bei 90% der positiven Klone entspricht (Fig. 2a, 2b, 2c). Die sortierten Zellen wurden zwecks weiterer Analyse expandiert. Wir haben festgestellt, dass die RT-PCR einer PCR aus Genom-DNA hinsichtlich der Isolierung integrierter cDNAs überlegen war. Eine nachfolgende Sequenzanalyse der Inserts aus positiven Klonen ergab die Identität der isolierten Klone, welche in Tab. 1 angeführt ist.

**Tab. 1: Beispiele für mittels vorliegenden Screening-Systems isolierte TFs:**

| NAME | Genbank Zugriffs-Nr. |
|---|---|
| MAML-1 | AF221759 |
| Notch-1 | AF308602 |
| HSF-1 | HUMHSF1 |
| Sox-7 | BC004299 |
| Eric-1 | HSA243997 |
| MondoA | AF312918 |
| E7 | XM_018098 |
| STAT6 | XM_043112 |
| Fus | HSFUSA |

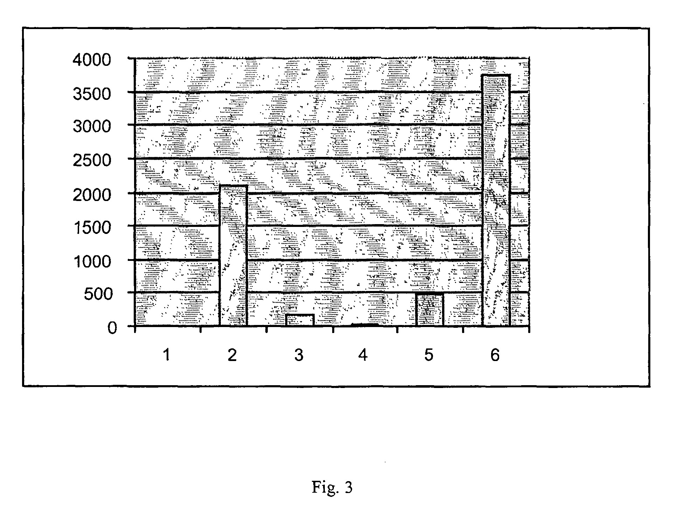

Analyse der TA-Domänen und Bestätigung der TA-Eigenschaften. Durch Datenbankabfragen ließ sich die Identität der isolierten Klone feststellen. Es wurde eine Reihe bekannter TFs ermittelt (siehe Tab. 1). Die Inserts aus selektierten Klonen wurden in den pBMN-Gal4-BstXI-Vektor kloniert und gemeinsam mit pFR-Luc (einem Gal4-abhängigen Promotor-Luciferase-Reporterkonstrukt von Stratagene) in Zellen transfiziert, um deren TA-Potential erneut zu bewerten. Die Ergebnisse sind in Fig. 3 dargestellt.

Fig. 3 bezieht sich auf den Luciferase-Test. Mittels RT-PCR gewonnene cDNA-Inserts wurden zurück in den pBMN-Gal4-BstXI Vektor subkloniert und vorübergehend gemeinsam mit dem Gal4-Luc-Reporter ebenso wie RSV-βgal als interne Kontrolle in NIH3T3-Zellen transfiziert. Die Luciferase-Werte wurden auf die β-Galaktosidase-Expression normalisiert. 1 (Negativkontrolle): leerer pBMN-Gal4-BstXI-Vektor; 2 (Positivkontrolle): NF-kB-p65-TA-Domäne in pBMN-Gal4-BstXI; 3: MAML-1; 4: STAT6; 5: E7; 6: Sox-7.

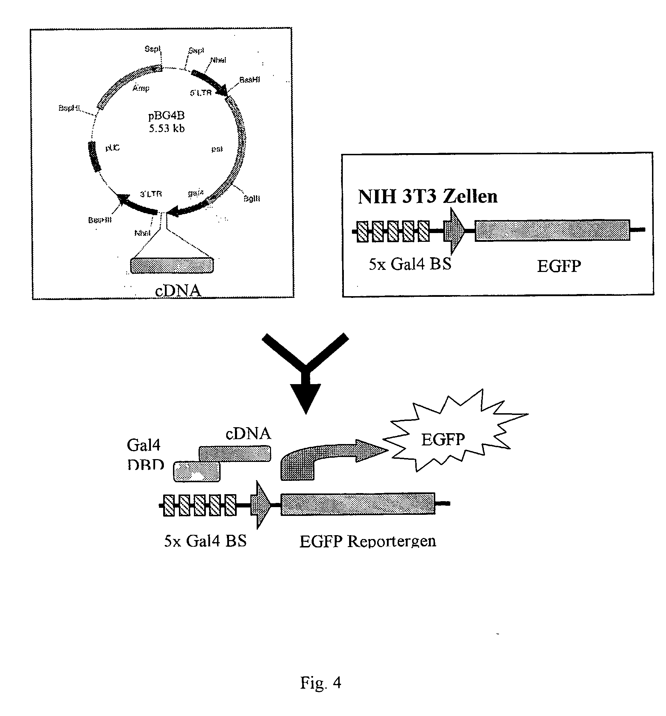

Fig. 4 zeigt eine schematische Darstellung des erfindungsgemäßen Screeningverfahrens.

Die folgenden Beispiele erläutern im Wesentlichen den Gegenstand der Erfindung:

### Beispiel 1:

Eine cDNA Bank aus mRNA von Zytokin-stimulierten (ein Mix aus TNFa, IL-1 und LPS, für die Dauer von 0, 2 und 6 Std. appliziert) Endothelzellen wird in dem Plasmid Vektor pBG4G generiert. Dieser Vektor ist ein Derivat des retroviralen Expressions Vektors pBABE und enthält zusätzlich die Hefe Gal4 DNA Bindungsdomäne in einer Weise dass cDNA Inserts als Fusionsproteine mit der gal4 DNA Bindungsdomäne exprimiert werden. Nach Verpackung in retrovirale Partikel wird die Bank in eine Reporterzelllinie transduziert. Diese Reporterzelllinie besteht aus NIH3T3 Zellen in welche ein Reportergen stabil integriert wurde. Das Reportergen besteht aus einem Gal4- abhängigen Promoter der die Expression von GFP kontrolliert. cDNA Inserts, welche eine DNA Bindungdomäne enthalten, bilden zusammen mit der Gal4 DNA Bindungsdomäne aus dem Vektor einen funktionellen Transkriptionsfaktor, welcher die Expression des Reportergens bewirkt, die sich fluoreszenzmikroskopisch oder mittels FACS nachweisen lässt. Zellen, die somit durch ihre Fluoreszenz detektiert werden, werden mittels FACS isoliert, expandiert, das cDNA Insert mittels RT-PCR isoliert und sequenziert.

### Beispiel 2:

Individuelle Klonzelllinien, die ein bestimmtes Fusionsprotein bestehend aus der Hefe Gal4 DNA Bindungsdomäne sowie der Transaktivierungsdomäne eines bestimmten Transkriptionsfaktors enthalten, lassen sich dazu verwenden, niedermolekulare Substanzen zu identifizieren welche die transaktivierende Aktivität dieses Transkriptionsfaktors beinflussen. Dazu wird die entsprechende Zelllinie mit chemischen Substanzen verschiedener Konzentration, z.B. aus einer kombinatorischen Bank, versetzt und nach einer geeigneten Zeit die Expression des Reportergens gemessen und mit der Expression vor Substanzzugabe verglichen.

### Beispiel 3:

Verschiedene Zelllinien, welche jede ein bestimmtes Fusionsprotein bestehend aus der Hefe Gal4 DNA Bindungsdomäne sowie der Transaktivierungsdomäne eines bestimmten Transkriptionsfaktors enthält, lassen sich dazu verwenden, um den Mechanismus der Wirkung einer niedermolekularen Substanz oder eines Arzneimittels aufzuklären. Dazu werden die verschiedene Zelllinien mit verschiedenen Konzentrationen der zu testenden Substanz versetzt und die Expression der Reportergene gemessen und mit der Expression vor Substanzzugabe verglichen.

### Beispiel 4:

Das Verfahren lässt sich dazu verwenden, um Transaktivierungsdomänen unterschiedlicher Stärke zu identifizieren. Transaktivierungsdomänen unterschiedlicher Stärke werden zur Generierung von künstlichen Transkriptionsfaktoren benötigt, welche bei gentherapeutischen Methoden zur Modulierung der Expression bestimmter Gene zum Einsatz kommen. Beispielsweise lassen sich mittels des beschriebenen Verfahrens isolierte Transaktivierungsdomänen als Fusionsproteine mit künstlichen Zink-Finger DNA Bindungs-Domänen kombinieren, welche an spezifische DNA Bindungsstellen in der Promoterregion der zu beeinflussenden Gene binden.

### Material und Methoden

**Konstruktion des retroviralen Vektors und der cDNA-Bibliothek.** Eine cDNA-Bibliothek wurde generiert aus poly(A+)-RNA, die aus humanen Nabelschnur Endothelzellen (HUVEC) isoliert wurde, welche 0, 3 und 9 Stunden lang mit einer Mischung aus TNFα, IL-1α und LPS stimuliert worden waren, und zwar unter Verwendung eines Oligo-Random-Priming-Kits (Stratagene), gefolgt von der Ligation von BstXI-Adaptern (Seed und Aruffo, 1987). Die auf Größe selektierte cDNA (>1,5 kb) wurde in den retroviralen Vektor pBABE ligiert (Morgenstern und Land, 1990), welcher durch Insertion der Hefe-Gal-DNA-BD gefolgt von einem Polylinker, der zwei nicht selbstkomplementäre BstXI-Bindungsstellen (pBMN-Gal4-BstXI) enthielt, modifiziert wurde. Die Ligation wurde in XL-10 elektrokompetente Zellen (Strategene) transformiert, was eine Bibliothek von 2,2x10⁷ unabhängigen Klonen ergab, wovon 90% Inserts mit einer durchschnittlichen Größe von 1,3 kb aufwiesen. Die Bibliothek wurde unter Einsatz der Phoenix eco Verpackungszelllinie in retrovirale Partikel verpackt (Hitoshi et al., 1998). Als Positivkontrolle wurde die NF-kB-p65-TA-Domäne (Schmitz et al., 1994) in denselben Vektor eingefügt.

Konstruktion einer Gal4-Promotor-abhängigen GFP-Reporter-Zelllinie. Murine NIH3T3-Zellen wurden mit einem Vektor (pFR-EGFP) transfiziert, der den Gal4-abhängigen Minimalpromotor aus pFR-Luc (Stratagene) mit EGFP enthielt. Die Zellen wurden mit Puromycin selektiert, und mehrere Klone wurden in transienten Transfektionen auf EGFP-Expression unter Verwendung des Gal4-NF-kB-TA-Konstrukts als Positivkontrolle getestet.

**Retrovirale Transduktion, FACS-Sortierung und Analyse.** Ein retroviraler Überstand wurde auf die Reporter-Zelllinie wie beschrieben aufgebracht, und EGFP-positive Klone wurden mittels FACS unter Verwendung eines FACS Vantage Sortiergeräts (Becton Dickinson Immunocytometry Systems, San Jose, CA, USA) direkt in 96 Well-Plates sortiert. Die RNA wurde 14 Tage später aus den einzelnen Klons isoliert und die Inserts mittels RT-PCR zurückgewonnen und sequenziert.

**Luciferase-Test.** PCR-Fragmente aus positiven Klonen wurden mit BstXI verdaut und in den pBMN-Gal4-BstXI eingefügt, und gemeinsam mit pFR-Luc und RSV-βgal als interne Kontrolle in Zellen transfiziert. Luciferase- und β-gal-Tests wurden wie beschrieben durchgeführt (de Wet et al., 1987).

## Patentansprüche

1. Verfahren zur spezifischen Identifikation von Transkriptionsfaktoren und Transkriptionskoaktivatoren aus einem bestimmten Zelltyp, **dadurch gekennzeichnet, dass** eine cDNA-Bibliothek in einem Vektor generiert wird, welche Inserts als Fusionsproteine mit der Hefe Gal4 DNA Bindungsdomäne exprimiert.

2. Verfahren nach Anspruch 1, **dadurch gekennzeichnet, dass** der verwendete Zelltyp Endothelzellen sind.

3. Verwendung des Verfahrens nach Anspruch 1 zur Identifikation starker Transaktivierungs-Domänen als Komponenten künstlicher Transskriptionsfaktoren.

## Claims

1. Method for the specific identification of transcription factors and transcription co-activators from a certain cell type, **characterised in that** a cDNA library, which expresses inserts as fusion proteins with the yeast Gal4 DNA binding domain, is generated in a vector.

2. Method according to claim 1, **characterised in that** the cell type used is endothelial cells.

3. Use of the method according to claim 1 for the identification of strong transactivation domains as components of artificial transcription factors.

## Revendications

1. Procédé pour l'identification spécifique de facteurs de transcription et de co-activateurs de transcription à partir d'un type cellulaire déterminé, **caractérisé en ce qu'**une banque d'ADNc est générée dans un vecteur qui exprime les inserts sous la forme de protéines de fusion avec le domaine de liaison à l'ADN Gal4 de la levure.

2. Procédé selon la revendication 1, **caractérisé en ce que** le type cellulaire utilisé est représenté par des cellules endothéliales.

3. Utilisation du procédé selon la revendication 1 pour identifier des domaines de transactivation forts comme composants de facteurs de transcription artificiels.
